# DEMANDE DE BREVET EUROPEEN

(11) **EP 1 958 640 A1**
(43) Date de publication de la demande: **20.08.2008**
(21) Numéro de dépôt: 08356025.0
(22) Date de dépôt: 14.02.2008
(51) Int. Cl.: A61K 33/10, C01F 7/00, C01F 7/14, C04B 22/10

(54) **Procédé de fabrication d'hydroxycarbonate d'aluminium**

(30) Priorité: 16.02.2007 FR 0701147
(71) Demandeur: Duclos Chimie, 13240 Septemes Les Vallons (FR)
(72) Inventeur: Margnat, Jacques, 13240 Septemes Les Vallons (FR); Margnat, Frank, 13008 Marseille (FR); Meric, Pascal, 30128 Garons (FR)
(74) Mandataire: Maureau, Philippe

(57) **Abrégé**

La présente invention concerne un procédé de fabrication d'hydroxycarbonate d'aluminium, caractérisé en ce qu'il comporte les étapes suivantes :
a) mise en solution de bicarbonate de sodium dans de l'eau pour obtenir une solution dont le pH est compris entre 8 et 9,
b) ajout dans la solution obtenue à l'étape a, d'une solution d'aluminate de sodium,
c) ajout, simultané à l'ajout de l'étape b), dans la solution obtenue à l'étape a), d'une solution de sulfate d'aluminium, le pH du mélange ainsi obtenu étant maintenu à une valeur de 8,0 ± 0,1 jusqu'à la fin de la précipitation de l'hydroxycarbonate de sodium.

Elle concerne également ledit procédé caractérisé en ce qu'il comporte en outre une étape d), en fin de précipitation, au cours de laquelle, le pH du mélange est abaissé à 5,8.

## Description

La présente invention concerne un procédé de préparation d'hydroxycarbonate d'aluminium. L'invention concerne plus particulièrement un procédé pour la préparation d'un hydroxycarbonate d'aluminium, sous forme de slurry, utilisé dans la fabrication d'accélérateurs de prise pour agents liants tel que le ciment.

On connaît l'utilisation de l'hydroxyde d'aluminium et des sels d'aluminium dans la préparation d'accélérateurs de prise et de durcissement.
Par exemple, le brevet US 6 302 954 décrit un procédé de préparation d'un accélérateur de prise et de durcissement liquide. Le mélange réactionnel comprend au moins un sel basique d'aluminium et/ou de l'hydroxyde d'aluminium, au moins un sulfate d'aluminium et/ou de l'acide sulfurique, au moins un ou un mélange d'au moins deux acides carboxyliques organiques, des sels d'aluminium d'acides carboxyliques et au moins une substance organique et/ou inorganique choisie dans le groupe comprenant les sulfates, les hydrogénosulfates, les carbonates, les hydrogénocarbonates (ou bicarbonates), les oxydes de métaux alcalino-terreux et les hydroxydes de métaux alcalino-terreux. De l'eau est ajoutée à ce mélange et le tout est chauffé à 150°C afin d'obtenir un produit final sous forme aqueuse.
Le brevet US 4 507 154 décrit un procédé de fabrication d'une préparation, ne contenant pas d'ions métalliques alcalins ni de chlore, permettant l'accélération de la prise et du durcissement d'un agent hydraulique. Cet accélérateur contient de l'hydroxyde d'aluminium, au moins un sulfate, nitrate ou formiate de calcium, magnésium, fer ou aluminium, soluble dans l'eau, et au moins un polymère.
La demande de brevet WO 2001/30720 décrit la composition d'un accélérateur de prise, sous forme d'une suspension aqueuse, contenant un polyol, un sel ou un oxyde d'aluminium et de la silice.
La demande de brevet EP 1 095 922 décrit des accélérateurs de prise et de durcissement liquides pour liants hydrauliques. Ces accélérateurs sont sans ions alcalins ni chlore et contiennent des sels d'aluminium, des agents complexants, des inhibiteurs de corrosion et des agents épaississants ou thixotropiques.

L'inconvénient de ces différentes compositions est le fait de devoir dissoudre l'hydroxyde d'aluminium et les sels d'aluminium dans de l'eau lors de leur mise en oeuvre. Il y a alors un risque de floculation de la préparation.

L'utilisation d'un hydroxycarbonate d'aluminium sous forme de slurry permet de résoudre ce problème.
L'hydroxycarbonate d'aluminium peut facilement se préparer sous forme de slurry. Le slurry ainsi obtenu est un produit ayant des propriétés intéressantes pour la préparation d'accélérateurs de prise pour agents liants.

Un slurry d'hydroxycarbonate d'aluminium ou hydroxyde carbonate d'aluminium est une suspension aqueuse de particules insolubles d'hydroxyde d'aluminium sur lesquelles des anions carbonates sont adsorbés et complexés de façon intégrée à la structure polymère de l'hydroxyde d'aluminium.

L'utilisation d'un slurry d'hydroxycarbonate d'aluminium permet de s'affranchir des problèmes de mise en solution dans de l'eau que l'on aurait avec de l'hydroxyde d'alumine sec. L'avantage d'utiliser un tel slurry est qu'il est plus stable puisque déjà en suspension.

Les propriétés du slurry, qui sont d'intérêt pour le fabricant d'agents liants, sont sa viscosité et sa pureté, c'est-à-dire la quantité d'impuretés, telles qu'un sel ou un ion alcalin, présentes dans le slurry.
Les accélérateurs de prise à forte teneur en ions alcalins réduisent la stabilité finale du ciment et augmentent le rétrécissement, qui peut conduire à la formation de fissures, ce qui n'est pas admissible pour la pérennité des constructions.
La viscosité est une caractéristique importante car elle détermine la facilité de mise en oeuvre du slurry dans le procédé de fabrication de l'additif de prise rapide. Elle doit être faible pour éviter une sédimentation trop rapide du produit, chargé en alumine, lors de sa mise en oeuvre.

De nombreux procédés de préparation d'hydroxycarbonate d'aluminium ont été décrits. En effet, dans de nombreuses publications, l'hydroxycarbonate d'aluminium est également utilisé en pharmacie comme agent antiacide ou en tant que pansement gastrique. Il est bien entendu que lorsque les procédés concernent les applications pharmaceutiques, les contraintes de préparation et les propriétés attendues pour le produit final ne sont pas les mêmes, mais la teneur en cations métalliques restent cependant une propriété importante.

Par exemple, le brevet FR 2 575 454 décrit un procédé continu pour la fabrication d'un gel d'hydroxycarbonate d'aluminium, utilisé comme agent antiacide, ayant des valeurs prédéterminées de viscosité et de teneur en ions alcalins. Les réactifs utilisés sont un sel d'aluminium, un carbonate alcalin et un aluminate alcalin. L'aluminate de sodium est ajouté comme constituant de la solution de carbonate de sodium et constitue un ingrédient mineur du mélange. Le pH de la réaction est maintenu à une valeur prédéterminée comprise entre environ 5,90 et environ 6,10 (± environ 0,05); cette régulation est effectuée en faisant varier les quantités relatives des différents réactifs en jouant sur leurs débits d'entrée. Le gel obtenu a une teneur en sodium d'environ 0,19 à 0,25 mg/g et une viscosité d'environ 200 à environ 800 cP mesurée à une concentration de 8,5 % p/p d'Al2O3 avec un viscosimètre de Brookfield LVT, tige n°2, à 12 tr/min.

Le brevet US 495 087 décrit un procédé continu pour la fabrication d'un gel d'hydroxycarbonate d'aluminium, utilisé comme agent antiacide. Ce procédé consiste à faire réagir une solution aqueuse de sel d'aluminium avec une solution aqueuse alcaline contenant des anions carbonates et bicarbonates pour obtenir la précipitation d'un gel d'hydroxycarbonate d'aluminium. Le pH de la réaction est maintenu à une valeur prédéterminée pour contrôler la réaction d'hydrolyse et pour faciliter l'incorporation des anions carbonates dans la structures polymérique. Le système utilisé comporte deux réacteurs, le premier contenant le mélange maintenu à un pH compris entre environ 6,5 et environ 7,0 et le deuxième contenant le mélange à un pH compris entre environ 6,4 et environ 6,8. Le produit obtenu a une teneur en cations alcalins métalliques qui n'est pas supérieure à environ 0,5% en poids par rapport au poids du gel mouillé.

Le brevet US 3 911 090 décrit un procédé pour produire un hydroxycarbonate d'aluminium, utilisé comme agent antiacide, classique (c'est-à-dire réaction entre un sel soluble d'aluminium et une solution aqueuse basique contenant des ions carbonates et/ou bicarbonates) mais effectué en présence d'un silicate alcalin métallique. L'hydroxycarbonate d'aluminium se forme à un pH compris entre 6 et 8 et est tel qu'il ne perd pas ses propriétés antiacides s'il est stocké pendant une longue période.

Le brevet US 4 053 568 décrit une méthode pour préparer un hydroxycarbonate d'aluminium, utilisé comme agent antiacide, en ajoutant simultanément à de l'eau, une solution aqueuse de sulfate d'aluminium et une solution aqueuse de bicarbonate alcalin et/ou de carbonate alcalin, tout en maintenant le pH du mélange à une valeur comprise entre 6,8 et 7,8. Après précipitation complète, le pH du mélange est diminué jusqu'à une valeur de 6 grâce à un ajout d'une solution aqueuse d'au moins un sel d'acide fort d'aluminium. Le but de ce procédé est d'obtenir un gel ayant un pH presque neutre. En effet, si ce gel a un pH alcalin élevé, il est alors inutilisable comme antiacide car incompatible avec les conditions physiologiques de l'estomac.

Le problème de l'élimination des impuretés a été étudié dans le brevet US 4 500 444. Le produit préparé, selon ce document, est utilisé dans le domaine pharmaceutique, de l'hygiène et des cosmétiques. Il peut également être utilisé comme agent épaississant ou de suspension, comme émulsifiant, comme agent ancrant et comme élément permettant l'obtention de film sans graisse. Ce document présente la fabrication de gels de carbonate d'aluminium. La réaction se fait à un pH de 5,75 ou de 6,4 lorsque les réactifs sont l'aluminate de sodium, un sel d'aluminium acide et du carbonate de sodium. Le pH est contrôlé grâce à la variation du débit d'entrée d'un des réactifs. Les inventeurs indiquent que, lors de la préparation du gel de carbonate d'aluminium, l'élimination des ions sodium présente des difficultés. Ils solutionnent ce problème en maintenant le pH, au cours de la réaction, à une valeur prédéterminée acide ce qui entraîne une diminution de l'adsorption des impuretés sur le gel permettant un lavage et une filtration plus facile.

Le brevet US 3 395 221 décrit un procédé pour la production d'un gel d'hydroxyde d'aluminium, utilisable dans des préparations pharmaceutiques, qui, consiste à faire réagir un sel d'aluminium avec un hydroxyde de métal alcalin dans un milieu réactionnel aqueux. Le pH de la réaction est maintenu à un pH compris entre environ 8,2 et environ 8,9 grâce au contrôle des débits d'entrée des réactifs. Les inventeurs ont trouvé qu'une diminution du pH au cours de la réaction entraîne une augmentation de la teneur en impuretés solubles et qu'une augmentation du pH au cours de la réaction entraîne une diminution de la teneur en impuretés solubles.

Le procédé selon l'invention permet de produire un slurry d'hydroxycarbonate d'aluminium ayant une valeur de viscosité et une teneur en ions alcalins prédéterminées.
La teneur maximale en ions sodium admise est de 1,3% en poids et la viscosité du slurry obtenu doit être comprise entre 20 et 30 cP.

L'invention concerne un procédé de préparation d'hydroxycarbonate d'aluminium, caractérisé en ce qu'il comporte les étapes suivantes :
a) mise en solution de bicarbonate de sodium dans de l'eau pour obtenir une solution dont le pH est compris entre 8 et 9,
b) ajout dans la solution obtenue à l'étape a, d'une solution d'aluminate de sodium,
c) ajout, simultané à l'ajout de l'étape b, dans la solution obtenue à l'étape a, d'une solution de sulfate d'aluminium, le pH du mélange ainsi obtenu étant maintenu à une valeur de 8,0 ± 0,1 jusqu'à la fin de la précipitation de l'hydroxycarbonate de sodium.

Le procédé selon l'invention est un procédé discontinu. Il consiste en une méthode de précipitation mixte, dans un système fermé (batch) : le mélange, contenant le précipité, n'est évacué du réacteur qu'une fois que l'ensemble des réactifs a réagi. Le précipité est ensuite lavé et filtré jusqu'à obtenir le produit final souhaité.

Dans un mode de réalisation, le procédé comporte en outre une étape d), en fin de précipitation, au cours de laquelle, le pH du mélange est abaissé entre 5,7 et 6,1.

Dans un mode de réalisation, le pH du mélange est abaissé à 5,8.
Dans un mode de réalisation, le pH du mélange est abaissé par addition d'une solution de sulfate d'aluminium.

Dans un mode de réalisation le procédé comporte en outre une étape e) de filtration et une étape f) de lavage pour obtenir un hydroxycarbonate d'aluminium sous forme de slurry.

La précipitation et la formation d'hydroxycarbonate d'aluminium peuvent être obtenues classiquement par mise en présence d'un sel soluble d'aluminium et d'une solution aqueuse basique comportant des ions carbonates et/ou bicarbonates.

Dans le procédé selon l'invention, les réactifs utilisés sont précisément du bicarbonate de sodium, de l'aluminate de sodium et du sulfate d'aluminium.
Des sels de sodium sont utilisés, bien que la teneur en sodium dans le produit final soit un facteur limitatif, pour réguler le pH et le maintenir à environ 8.
Le bicarbonate de sodium en solution est utilisé comme source d'ions carbonates en solution basique en raison de ses propriétés tampon qui permettent de réguler et de stabiliser plus facilement le pH.
L'utilisation de l'aluminate de sodium permet également de mettre en oeuvre une source en ions aluminium qui soit fortement basique et de favoriser la formation de l'hydroxycarbonate d'aluminium.
Cependant pour éviter des fluctuations importantes du pH, une seconde source en ions aluminium est utilisée : une solution de sulfate d'aluminium, solution acide, est introduite simultanément à l'introduction de la solution d'aluminate de sodium.

Une régulation du pH à une valeur de 8, pendant toute la précipitation, permet de limiter la chute de viscosité du produit afin d'obtenir un gel final ayant la viscosité souhaitée.

Comme la quantité de bicarbonate mise en oeuvre est en excès, la régulation du pH, pendant toute la précipitation, est effectuée par régularisation des débits des solutions d'aluminate et/ou de sulfate d'aluminium.
Dans un mode de réalisation, le débit de sulfate d'aluminium est asservi au pH.
Dans un mode de réalisation, le maintien du pH du mélange obtenu à l'étape c) est effectué par contrôle du débit d'entrée de la solution de sulfate d'aluminium, le débit d'entrée de la solution d'aluminate de sodium étant constant.

En fin de précipitation, selon un mode de réalisation, le pH du milieu réactionnel est abaissé pour limiter l'absorption des impuretés dans l'hydroxycarbonate d'aluminium obtenu dans l'étape d) dudit procédé.
Cette diminution du pH doit être faite après la fin de la précipitation de l'hydroxycarbonate d'aluminium afin de ne pas avoir une trop forte chute en viscosité du produit final.
Si le pH n'est pas diminué en dessous de 6,1, le produit final contiendra trop d'impuretés (notamment du sodium). Mais si le pH est trop abaissé (en dessous de 5,7), on aboutit à la décarbonatation du produit et à une viscosité inférieure à celle souhaitée pour le produit final.
Dans un mode de réalisation, le pH est abaissé à 5,8.
Dans un mode de réalisation, le pH est abaissé par addition d'une solution acide.
Dans un mode de réalisation, le pH est abaissé par addition d'un excès de solution de sulfate d'aluminium.
Le débit de la solution de sulfate d'aluminium est asservi au pH du mélange réactionnel.
Dans un mode de réalisation, le débit d'entrée de la solution de sulfate d'aluminium est constant.

Tout le processus de préparation jusqu'au transfert du produit dans les cuves pour lavage et filtration est conduit sous agitation mécanique violente car de l'agitation dépend également la stabilité de l'hydroxycarbonate après synthèse. Cette agitation violente permet d'obtenir une défloculation acceptable de l'hydroxycarbonate et donc de ralentir le processus de sédimentation ou caking du produit.

Après obtention du précipité d'hydroxycarbonate d'aluminium, le procédé mis en oeuvre comporte en outre une étape e) de filtration et une étape f) de lavage qui permettent d'obtenir l'hydroxycarbonate d'aluminium sous forme de slurry.
L'étape de filtration est conduite par la mise en oeuvre des techniques classiques de filtration connues.
L'étape de lavage est effectuée pour éliminer les sels en excès et notamment les sels de sodium.
Compte tenu de l'utilisation d'excès de sulfate d'aluminium, les sels de sodium, devant être éliminés, sont sous forme principalement de sulfate de sodium.
Les lavages sont effectués avec de l'eau.
Une fois le produit filtré et lavé, la pâte est transférée dans un défloculeur. Le passage dans ce défloculeur a pour effet de reliquéfier le produit grâce à ses propriétés thixotropes. En sortie de défloculeur, un produit final liquide, ayant une viscosité très faible, est obtenu.
L'humidité du produit est, en fin de processus de préparation, d'environ 60 ± 3 %.

Le pH est mesuré par les techniques classiques connues de l'homme de l'art.
Dans un mode de réalisation, il est mesuré par une sonde à compensation de température. Cette sonde de pH est placée dans un bol se trouvant dans le circuit de recirculation du produit, dispositif intégré aux cuves de préparation.

Les mesures de viscosité finale du slurry se font en mesurant la propriété de résistance à l'écoulement uniforme sans turbulence se produisant dans le produit final. Ces mesures sont effectuées sur un appareil viscosimètre de marque Brookfield (ref. LVDVE) dont le corps et la vitesse sont adaptées à la plage de viscosité mesurée. Le corps utilisé est de type 3 et la vitesse de 100 tours/min.

Les mesures des teneurs en sodium sont effectuées au moyen d'un spectromètre de flamme sur des échantillons pris en fin de préparation, après filtration et lavage.

L'invention concerne également un hydroxycarbonate d'aluminium susceptible d'être obtenu par le procédé selon l'invention, tel que décrit ci-dessus, et caractérisé en ce que sa teneur en ions sodium est inférieure à 1,3%.
Elle concerne également un hydrocarbonate d'aluminium tel que précédemment défini, caractérisé en ce qu'il se présente sous forme d'un slurry dont la viscosité est comprise entre 20 et 30 cP.
Elle concerne également un hydrocarbonate d'aluminium tel que précédemment défini, caractérisé en ce que le rapport molaire CO₂/Al₂O₃ est compris entre 0,17 et 0,24.
Elle concerne plus particulièrement un hydrocarbonate d'aluminium tel que précédemment défini, caractérisé en ce que le rapport molaire CO₂/Al₂O₃ est de 0,2.

Le procédé selon l'invention comporte cinq étapes bien distinctes.
La première étape, étape a), consiste en une dissolution de bicarbonate de sodium dans de l'eau, permettant d'obtenir une solution aqueuse ayant un pH compris entre 8 et 9.
Lors des étapes b) et c) du procédé, une solution aqueuse d'aluminate de sodium et une solution aqueuse de sulfate d'aluminium sont ajoutées simultanément à la solution aqueuse de bicarbonate de sodium. C'est au cours de ces deux étapes qu'est opérée une précipitation double de l'hydroxycarbonate d'aluminium. Le pH du mélange est maintenu à une valeur de 8,0 ± 0,1, pendant toute la durée de la précipitation.
Dans un mode de réalisation, le maintien du pH du mélange obtenu à l'étape c) est effectué par contrôle du débit d'entrée de la solution de sulfate d'aluminium qui est régulé en fonction du pH, le débit d'entrée de la solution d'aluminate de sodium étant constant.

La précipitation de l'hydroxycarbonate d'aluminium est instantanée. La réaction se fait avec un excès de bicarbonate de soude, elle est donc terminée lorsque tout l'aluminate de soude a été ajouté.
Dans un mode de réalisation, le procédé comporte en outre une étape d), au cours de laquelle une solution aqueuse de sulfate d'aluminium est ajoutée au mélange réactionnel, en fin de précipitation, pour abaisser le pH à une valeur comprise entre 5,7 et 6,1.
Dans un mode de réalisation elle est ajoutée jusqu'à ce que le pH du mélange soit de 5,8.
Dans un mode de réalisation, le débit d'entrée de la solution de sulfate d'aluminium, destiné à faire diminuer le pH dans l'étape d) du procédé, est constant.
Le précipité d'hydroxycarbonate d'aluminium obtenu est ensuite filtré, lors de l'étape e), et lavé, lors de l'étape f), afin d'obtenir un hydroxycarbonate d'aluminium sous forme de slurry.
Selon l'invention, une seule étape filtration/lavage est nécessaire pour permettre d'obtenir un taux final en sodium inférieur à 1,3%.

L'invention sera mieux comprise à la lecture de l'exemple qui suit.

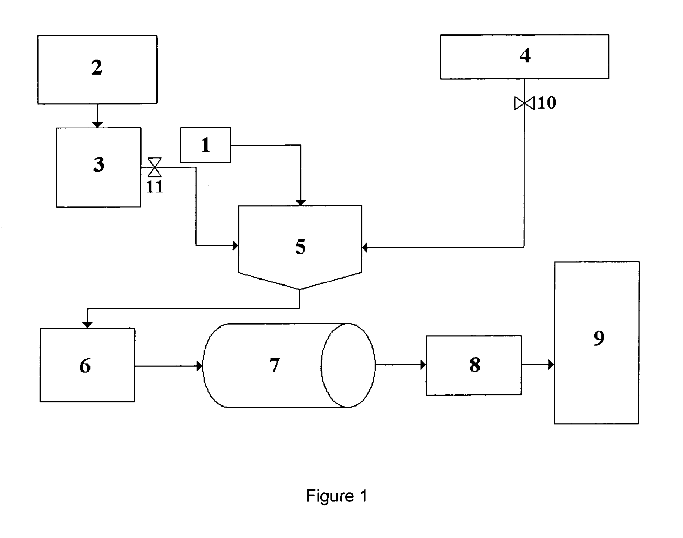

La figure 1 illustre schématiquement un mode de réalisation de l'installation utilisée pour la production, de type « batch » de l'hydroxycarbonate d'aluminium sous forme de slurry.

On dispose d'un réacteur (5) et d'une cuve (1) destinée au stockage de l'eau.
Le sulfate d'aluminium est stocké dans une cuve (4) qui comporte à sa sortie une vanne (10) permettant de réguler le débit.
La solution concentrée d'aluminate de sodium est stockée dans une cuve (2) qui comporte une cuve tampon (3) et une vanne (11) permettant de réguler le débit.
A la sortie du réacteur, une cuve (6) permet de stocker le produit avant son passage sur le filtre.
Un filtre rotatif (7), permettant d'effectuer l'isolement du gâteau obtenu, est équipé en sortie d'un défloculeur (8) et d'une cuve de stockage (9).

Lors de l'étape a), il est procédé à la dissolution dans le réacteur (5) de 290 kg de bicarbonate de sodium par addition de 2690 L d'eau. Le pH de la solution est compris ente 8 et 9.
Ensuite, 1946 L d'une solution d'aluminate de sodium concentrée à un débit constant de 1,2 m³/h (étape b) et une solution de sulfate d'aluminium (étape c) sont ajoutées à la solution de bicarbonate de sodium. Le débit d'entrée de la solution de sulfate d'aluminium est régulé à une valeur de consigne comprise entre 2,0± 0,2 m³/h en fonction du pH; à un instant donné la valeur exacte de son débit est fonction du pH du mélange réactionnel.
On observe, au cours de ces deux étapes, une précipitation. Au fur et à mesure que la quantité en réactifs augmente, un précipité d'hydroxycarbonate d'aluminium se forme en suspension dans la phase liquide du mélange réactionnel.

En fin de précipitation, lors d'une troisième étape (étape d), une solution de sulfate d'aluminium est ajoutée, à un débit constant de 1,6 m³/h, au mélange réactionnel afin de faire chuter le pH à une valeur de 5,8.

Lors de la descente du pH de 8 à 5,8, la viscosité du mélange réactionnel diminue, passant de 50 cP à 24 cP.

La filtration du précipité obtenu (étape e) et son lavage (étape f) sont effectués à l'aide d'un filtre rotatif de surface 50 m² et d'un média filtrant de type toile polypropylène de perméabilité 10 L/dm²/min. Le filtre rotatif fonctionne de façon continue ; il est de type extractif, la filtration étant effectuée sous vide.
La filtration sur le filtre rotatif permet de séparer la phase solide de la phase liquide.
Pendant la filtration, plusieurs opérations se succèdent.
Les 3 premières sont effectuées sous vide et consistent en :
- l'immersion et l'application du gâteau sur la toile filtrante
- la sortie d'immersion, le lavage et l'évacuation des filtrats et des effluents
- l'essorage, la concentration du gâteau et l'élimination des effluents

La quatrième et dernière opération est effectuée dans le secteur soufflé ; il s'agit de l'extraction du gâteau.

Une seule opération filtration/lavage est nécessaire pour permettre d'obtenir un taux final en sodium inférieur à 1,3%.
Avant lavage, donc à pH 5,8, le taux de sodium est de 4,7%. Après filtration et lavage, le taux final en sodium est de 0,26%.

La phase liquide filtrée est particulièrement chargée en Na₂SO₄, tandis que la phase solide, également appelé gâteau, contient environ 20% d'Al₂O₃. Le lavage permet quant à lui d'éliminer les impuretés encore présentes dans le gâteau.

## Revendications

1. Procédé de préparation d'hydroxycarbonate d'aluminium, **caractérisé en ce qu'**il comporte les étapes suivantes :
a) mise en solution de bicarbonate de sodium dans de l'eau pour obtenir une solution dont le pH est compris entre 8 et 9,
b) ajout dans la solution obtenue à l'étape a, d'une solution d'aluminate de sodium,
c) ajout, simultané à l'ajout de l'étape b), dans la solution obtenue à l'étape a), d'une solution de sulfate d'aluminium, le pH du mélange ainsi obtenu étant maintenu à une valeur de 8,0 ± 0,1 jusqu'à la fin de la précipitation de l'hydroxycarbonate de sodium.

2. Procédé de préparation d'hydroxycarbonate d'aluminium selon la revendication 1, **caractérisé en ce que** le maintien du pH du mélange obtenu à l'étape c) est effectué par contrôle du débit d'entrée de la solution de sulfate d'aluminium, le débit d'entrée de la solution d'aluminate de sodium étant constant.

3. Procédé de préparation d'hydroxycarbonate d'aluminium selon les revendications 1 et 2, **caractérisé en ce qu'**il comporte en outre une étape d), en fin de précipitation, au cours de laquelle, le pH du mélange est abaissé entre 5,7 et 6,1.

4. Procédé de préparation d'hydroxycarbonate d'aluminium selon la revendication 3, **caractérisé en ce que** le pH du mélange est abaissé à 5,8.

5. Procédé de préparation d'hydroxycarbonate d'aluminium selon les revendications 3 et 4, **caractérisé en ce que** le pH du mélange est abaissé par addition d'une solution de sulfate d'aluminium.

6. Procédé de préparation d'hydroxycarbonate d'aluminium selon les revendications 3 à 5, **caractérisé en ce que** le débit d'entrée de la solution de sulfate d'aluminium est constant.

7. Procédé de préparation d'hydroxycarbonate d'aluminium selon les revendications 1 à 6, **caractérisé en ce qu'**il comporte en outre une étape e) de filtration et une étape f) de lavage pour obtenir un hydroxycarbonate d'aluminium sous forme de slurry.

8. Hydroxycarbonate d'aluminium susceptible d'être obtenu par un procédé selon les revendications 1 à 7, **caractérisé en ce qu'**il se présente sous forme d'un slurry dont la viscosité est comprise entre 20 et 30 cP.

9. Hydroxycarbonate d'aluminium susceptible d'être obtenu par un procédé selon les revendications 1 à 7, **caractérisé en ce que** le rapport molaire CO₂/Al₂O₃ est compris entre 0,17 et 0,24.

10. Hydroxycarbonate d'aluminium selon la revendication 9, **caractérisé en ce que** le rapport molaire CO₂/Al₂O₃ est de 0,2.

11. Hydroxycarbonate d'aluminium selon l'une quelconque des revendications 8 à 10, **caractérisé en ce que** sa teneur en ion sodium est inférieure à 1,3%.
